# DEMANDE DE BREVET EUROPEEN

(11) **EP 1 609 439 A1**
(43) Date de publication de la demande: **28.12.2005**
(21) Numéro de dépôt: 05356101.5
(22) Date de dépôt: 10.06.2005
(51) Int. Cl.: A61F 2/00

(54) **Implant pour le traitement de la rectocèle ou de la cystocèle**

(30) Priorité: 11.06.2004 FR 0406352
(71) Demandeur: Analytic Biosurgical Solutions - ABISS, 42000 St Etienne (FR)
(72) Inventeur: Delorme, Emmanuel, 71100 Chalon sur Saone (FR); Beraud, Jean-Marc, 42100 St Etienne (FR)
(74) Mandataire: Le Cacheux, Samuel L.R.

(57) **Abrégé**

Implant, pour le traitement de la cystocèle, de la rectocèle et/ou du prolapsus du dôme vaginal, présentant une structure mince, souple et comprenant un corps de support (**2**) à partir duquel s'étendent au moins :
■ deux stabilisateurs de suspension supérieurs (**3**),
■ et deux stabilisateurs de suspension inférieurs (**4**), disposés de part et d'autre du plan sagittal (**S**),
caractérisé en ce que chaque stabilisateur de suspension (**3, 4**) est réalisé de manière à présenter une faible extensibilité parallèlement à son axe longitudinal.

## Description

La présente invention concerne le domaine technique du traitement de la cystocèle et des prolapsus rectaux, notamment chez la femme âgée.

Les phénomènes de prolapsus résultent, généralement, d'un relâchement des tissus de suspension des organes génitaux ou rectaux, entraînant des troubles qui nécessitent une intervention chirurgicale.

Ainsi, il a été proposé des tentatives de reconstruction du système naturel de suspension des organes, affectés par le prolapsus, en mettant en oeuvre des sutures non résorbables ou des bandelettes de renfort. Cependant, ces techniques n'ont pas toujours donné satisfaction, notamment en raison du recours à une intervention chirurgicale lourde, entraînant une dissection de régions anatomiques non concernées par la réparation chirurgicale, pour la réalisation des points de sutures non résorbables.

Afin de tenter de remédier à ces inconvénients, une demande de brevet FR 2 785 521 a proposé de mettre en oeuvre un implant comprenant un corps de support, à partir duquel s'étendent deux cordons de suspension, pourvus, à leurs extrémités, de pièces d'ancrage destinées à faire l'objet de sutures sur des zones réputées pour être anatomiquement stables. Cet implant est alors mis en place par voie laparoscopique permettant d'alléger la procédure chirurgicale

Cependant, il est apparu qu'un tel implant n'est pas en mesure d'assurer une suspension efficace, en raison, notamment, des contraintes appliquées aux zones réputées pour être anatomiquement stables. De plus, ce type d'implants ne présente pas, en condition d'utilisation, une grande stabilité spatiale.

Ainsi, il est apparu le besoin, d'une part, de disposer d'un implant offrant une meilleure stabilité d'implantation et, d'autre part, d'une technique qui permette d'obtenir une stabilité optimale, tout en réduisant au maximum le traumatisme subi par la patiente.

Ainsi, afin d'atteindre ces objectifs, il est proposé un implant pour le traitement de la rectocèle ou de la cystocèle, présentant une structure mince et souple et comprenant un corps de support à partir duquel s'étendent au moins deux stabilisateurs de suspension supérieure disposées de part et d'autre d'un plan sagittal du corps de support et deux stabilisateurs de suspension inférieurs disposées, également, de part et d'autre du plan sagittal.

Ce nouvel implant donne pleinement satisfaction, toutefois il est apparu encore possible de l'améliorer. Effet, il est apparu que dans certaines configuration d'utilisation, une traction importante appliquée sur les stabilisateurs de suspension induit une déformation indésirable et gênante desdits stabilisateurs de suspension de sorte qu'il est difficile d'assurer rapidement une mise en place sans tension résiduelle des stabilisateurs ou de conserver une configuration adaptée du corps de l'implant ou encore de contrôler la conformation d'implantation de l'ensemble de l'implant ou prothèse.

Afin d'apporter une solution à ce problème, l'invention concerne un implant, pour le traitement de la cystocèle, de la rectocèle et/ou du prolapsus du dôme vaginal, présentant une structure mince, souple et comprenant un corps de support à partir duquel s'étendent au moins :
■ deux stabilisateurs de suspension supérieurs,
■ et deux stabilisateurs de suspension inférieurs, disposés de part et d'autre du plan sagittal.

Selon l'invention cet implant est caractérisé en ce que chaque stabilisateur de suspension est réalisé de manière à présenter une faible extensibilité parallèlement à son axe longitudinal.

En effet, les inventeurs ont eu le mérite de déterminer que les déformations intempestives de l'implant et notamment des stabilisateurs de suspension pouvaient trouver leur origine dans les caractéristiques mécaniques du matériaux constitutif de l'implant notamment lorsque ce dernier est découpé dans une seule et même plaque de matériau biocompatible tissé ou tricoté de sorte que l'axe des stabilisateurs de suspension n'est pas parallèle à l'une ou l'autre des directions privilégiées de résistance à la traction.

Les inventeurs ont, en outre, déterminé que le meilleur comportement de l'implant était obtenu lorsque le corps de support est constitué dans un matériau prothétique relativement souple et extensible, tandis que les stabilisateurs de suspension sur lesquels s'exercera la force de traction de l'opérateur sont réalisés dans un matériau prothétique relativement peu extensible, afin de limiter leur déformation et leur permettre de conserver une conformation « à plat » et ainsi permettre à l'opérateur d'effectuer un réglage de la « tension » de montage plus fin qu'avec un matériau prothétique trop élastique.

Ainsi selon une caractéristique préférée mais non strictement nécessaire de réalisation de l'invention, les stabilisateurs de suspension présentent une extensibilité parallèlement à leur axe longitudinal inférieure à l'extensibilité du corps de l'implant selon notamment ses deux axes longitudinal et transversal.

Selon l'invention, il est possible d'obtenir ces différences de comportement à la traction du corps de support et des stabilisateurs de suspension de différentes manières.

Ainsi selon une forme de réalisation, l'ensemble de l'implant conforme à l'invention est tricoté en une seule pièce, mais il est adopté, pour le corps et les stabilisateurs de suspension, une armure et une orientation des mailles différentes, de manière que les stabilisateurs de suspension présentent une faible extensibilité longitudinale et, de préférence, inférieure à celle du corps de l'implant.

Selon une autre forme de réalisation, ce comportement différentiel est obtenu en venant rapporter chaque stabilisateur de suspension sur le corps de support. Il est ainsi possible d'orienter le matériau constitutif de chacun des stabilisateurs de suspension de manière à obtenir le comportement mécanique recherché.

Selon une caractéristique de l'invention, afin de faciliter la fixation des stabilisateurs de suspension sur le corps de support, ce dernier présente une patte d'attache pour chaque stabilisateur de suspension.

La mise en place et la fixation des stabilisateurs de suspension peut alors être réalisée de différentes façons.

Selon une caractéristique de l'invention, chaque stabilisateur de suspension est cousu sur le corps de support.

Selon une autre caractéristique de l'invention, chaque stabilisateur de suspension est collé sur le corps de support.

Selon encore une autre caractéristique de l'invention, chaque stabilisateur de suspension est soudé sur le corps de support.

Bien entendu, il pourrait être envisagé de combiner ces modes d'assemblage pour la réalisation d'implants selon l'invention.

Selon l'invention chaque stabilisateur de suspension peut être réalisé dans différents types de matériaux biocompatibles, résorbables ou pas, synthétiques ou d'origine animale et/ou végétale. Parmi les matériaux synthétiques biocompatibles utilisables, il est possible de citer les polyesters, le polypropylène ou, encore, les polyamides. De même, parmi les matériaux biocompatibles d'origine animale, il est possible de citer le collagène porcin transformé.

Le matériau constitutif des stabilisateurs de suspension peut alors présenter différents types de structures en étant, par exemple, constitué de fils ou fibres tissées ou tricotées ou, encore, de fibres assemblées pour former un matériau fibreux non-tissé. Le matériau constitutif des stabilisateurs de suspension pourrait, également, être formé d'un film ou d'un complexe de films de matières biocompatibles.

Selon une forme de réalisation, chaque stabilisateur de suspension est réalisé dans un matériau tricoté dont les fils de trame ou les fils de chaîne sont disposés parallèlement à l'axe longitudinal du stabilisateur de suspension.

De manière préférée, chaque stabilisateur de suspension est alors réalisé dans un tricot peu extensible, parallèlement à l'axe longitudinal du stabilisateur de suspension.

Dans une autre forme de réalisation, chaque stabilisateur de suspension est réalisé dans une nappe de matériau non tissé, présentant une résistance à la traction isotrope dans son plan d'extension.

Selon l'invention le corps de support de l'implant peut présenter différentes formes, telles que, par exemple, triangulaire, ovale, voire circulaire. Dans une forme de réalisation préférée mais non exclusive de réalisation, le corps de support présente une forme générale sensiblement rectangulaire avec une longueur comprise entre 60 et 90 mm et une largeur comprise entre 40 et 60 mm, sans que ces dimensions ne soient limitatives.

Bien entendu selon l'invention, il est possible d'envisager différentes conformations ou configurations pour les stabilisateurs de suspension et le corps de support.

Ainsi, dans une forme de réalisation, les axes longitudinaux des stabilisateurs supérieurs forment, entre eux, un angle supérieur à 45° et, de manière préférée mais non strictement nécessaire, un angle α compris entre 100° et 180° et, de préférence, entre 115° et 170°. Il est à noter que, selon une forme de réalisation préférée, le plan sagittal constitue un axe de symétrie de l'implant et donc bissecteur de l'angle α.

La mise en oeuvre de stabilisateurs de suspension supérieurs, présentant une telle orientation relative, permet de placer judicieusement ces dernières dans la région trans-sacrosciatique pour assurer une bonne répartition des efforts subis par le corps de support sur les points d'ancrage anatomiques des stabilisateurs, tout en garantissant la meilleure orientation spatiale du corps du support implanté chez la patiente.

Selon une caractéristique préférée mais non strictement nécessaire de l'invention, les stabilisateurs de suspension supérieurs et/ou inférieurs présentent une longueur supérieure à 100 mm et, de préférence, supérieure ou égale à 120 mm. Une telle longueur permet une bonne extension des stabilisateurs de suspension dans leurs zones d'insertion respectives et met à profit les frictions entre les bras ou stabilisateurs de suspension et les tissus traversés pour assurer le maintien de l'implant.

Selon une autre caractéristique de l'invention, les axes longitudinaux des stabilisateurs inférieurs forment, entre eux, de préférence mais non nécessairement, un angle β non nul. Ainsi, il doit être considéré que les stabilisateurs inférieurs ne sont pas parallèles entre eux. De manière préférée mais non strictement nécessaire, l'angle β est supérieur à 10° pour être, de préférence, compris entre 10° et 75° ou, encore, entre 100° et 180°, selon la pathologie devant être traitée.

Selon encore une autre caractéristique de l'invention, préférée mais non strictement nécessaire, le corps de support présente une forme générale sensiblement rectangulaire. De manière préférée mais non strictement nécessaire, le corps de support présente alors une longueur comprise entre 60 mm et 90 mm et une largeur comprise entre 40 mm et 60 mm.

Selon une forme de réalisation de l'invention, les stabilisateurs supérieurs s'étendent sensiblement à partir des coins supérieurs du corps de support et les stabilisateurs inférieurs s'étendent à partir des coins inférieurs du corps de support.

Selon une autre forme de réalisation de l'invention, les stabilisateurs supérieurs s'étendent sensiblement à partir des coins supérieurs du corps de support, tandis que les stabilisateurs inférieurs s'étendent à partir des deux grands côtés du corps de support. Chacun des stabilisateurs inférieurs s'étend alors, de préférence mais non nécessairement, à une distance du bord supérieur du corps de support valant entre 60 % et 87 % de la longueur du corps de support.

Selon une autre caractéristique de l'invention les stabilisateurs inférieurs présentent une zone élargie au niveau de leur raccordement avec le corps de support de l'implant.

Diverses autres caractéristiques de l'invention ressortent de la description ci-dessous effectuée en référence aux dessins annexés qui illustrent différentes formes de réalisation d'un implant selon l'invention, ainsi que de dispositifs introducteurs permettant de faciliter la mise en place dudit implant.

Par ailleurs, il doit être noté que les différentes caractéristiques de l'invention, décrites précédemment et ci-après, peuvent être combinées ensemble selon différentes variantes, en fonction de la pathologie à traiter.
La **fig. 1** est une élévation, en vue à plat, d'un implant selon l'invention destiné au traitement de la rectocèle.
La **fig. 2** est une élévation, en vue à plat éclatée de l'implant, tel qu'illustré à la **fig.1.**
La **fig. 3** est une élévation, partiellement arrachée, d'un guide perforateur pouvant être utilisé pour la mise en place de l'implant selon l'invention et présentant une forme arquée.
La **fig. 4** est une élévation d'une autre forme de réalisation d'un guide perforateur selon l'invention, présentant une extrémité d'introduction de forme hélicoïdale.
La **fig. 5** est une vue de gauche du guide perforateur selon la **fig. 4**.
La **fig. 6** est une vue de dessous du perforateur illustré à la **fig. 4.**
La **fig. 7** est une vue d'un introducteur selon l'invention, permettant la mise en place de l'implant illustré à la **fig. 1.**
La **fig. 8** est une vue, analogue à la **fig. 1,** montrant une variante de réalisation de l'implant selon l'invention.
La **fig. 9** est une vue d'un introducteur selon l'invention, permettant la mise en place de l'implant illustré à la **fig. 8.**
Les **fig. 10** et **11** sont des vues, analogues à la **fig. 1,** montrant des variantes de réalisation d'un implant selon l'invention.
La **fig. 12** est une vue anatomique montrant exemple d'implantation chez une femme d'un implant selon la **fig. 11.**

L'invention propose un implant plus particulièrement conçu pour le traitement de la rectocèle et désigné dans son ensemble par la référence **1** à la **fig. .1**. Cet implant **1** présente une structure mince et souple et se trouve réalisé dans un matériau biocompatible adapté, tel que, par exemple, un matériau synthétique, tissé ou non, ou, encore, tricoté, à base de fibres de polypropylène ou de polyester. Un tel matériau synthétique pourra alors être enduit ou non de produits favorisant la croissance cellulaire. De même, l'implant selon l'invention, pourrait être réalisé en matériaux naturels, tels que du « fascia latta » ou, encore, tout matériel biologique ou synthétique résorbable.

Conformément à l'invention, l'implant **1** comprend un corps de support **2** à partir duquel s'étendent deux stabilisateurs de suspension supérieurs **3,** disposés de part et d'autre d'un plan sagittal **S**. L'implant comprend, également, deux stabilisateurs de suspension inférieurs **4**, disposés aussi de part et d'autre du plan sagittal **S**. ces stabilisateurs agissent par la striction réalisée par les masse musculaires

Selon l'exemple illustré, le corps de support **2** présente une forme sensiblement rectangulaire, sans qu'une telle forme puisse être considérée comme strictement nécessaire au sens de l'invention, et les stabilisateurs de suspension supérieurs **3** s'étendent chacune à partir d'un coin supérieur du corps **2**. Les stabilisateurs de suspension inférieurs **4** s'étendent quant à eux chacun à partir d'un côté du corps de support **2** de forme rectangulaire.

Ainsi, compte tenu de la disposition des stabilisateurs de suspension inférieurs **4** il existe une sorte de petit tablier inférieur **9** au niveau du corps **2** de l'implant **1**.

Selon l'exemple illustré, les stabilisateurs de suspension inférieurs **4** prennent naissance sur un côté du corps de support **2**, à une distance **d** du bord supérieur **30** du corps de support **2,** valant, de manière préférée mais non strictement nécessaire, entre 60 % et 87 % de la longueur **L**_{**2**} du corps de support.

De façon préférée, le corps de support **2** est choisi pour présenter une longueur **L**_{**2**} comprise entre 60 mm et 90 mm et une largeur ***l***_{***2***} comprise entre 40 mm et 60 mm.

Selon une caractéristique préférée de l'invention, les axes longitudinaux **A**_{**3**} des stabilisateurs supérieurs **3** forment un angle α supérieur à 45°, de préférence compris entre 100° et 180° en étant, de manière plus particulièrement préférée, compris entre 115° et 170°.

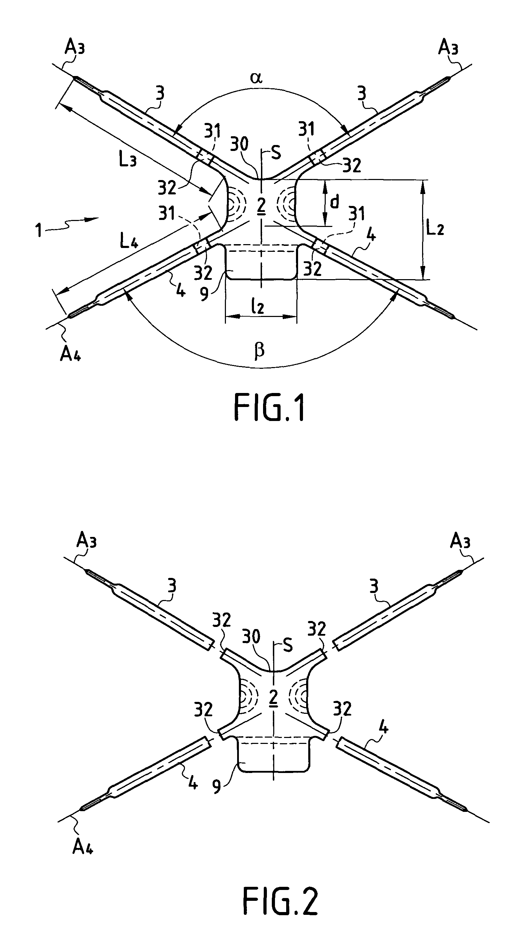

Par ailleurs, selon l'exemple de réalisation illustré à la **fig. 1,** les axes **A**_{**4**} des stabilisateurs de suspension inférieurs **4** forment entre eux un angle β, non nul, de préférence supérieur à 10° et, selon cet exemple de réalisation, un angle β compris entre 100° et 180°. Il est à noter que, de manière préférée, le plan sagittal **S** correspond à un plan de symétrie de l'implant **1** et se trouve donc bissecteur des angles α et β.

Afin de permettre une mise en place aisée des stabilisateurs de suspension dans leur zone d'insertion correspondante, tout en offrant une surface optimale de frottement avec les tissus traversés, les stabilisateurs de suspension supérieurs **3** et inférieurs **4** présentent une longueur respectivement **L**_{**3**}**, L**_{**4**} mesurée entre l'extrémité distale de chaque stabilisateur de suspension et le corps de support **2,** de préférence mais non nécessairement, supérieure à 100 mm et, de manière plus particulièrement préférée, supérieure à 120 mm. Les stabilisateurs de suspension présentent de plus une largeur comprise, de préférence mais non exclusivement, entre 5 mm et 15 mm et valant, par exemple, une dizaine de millimètres.

Afin d'éviter une déformation des stabilisateurs de suspension sous l'effet de la traction qui leur sera appliquée lors de la mise en place de l'implant, les stabilisateurs de suspensions sont réalisés de manière à présenter une faible extensibilité parallèlement à leur axe longitudinal **A**_{**3**}**, A**_{**4**}**.** A cet effet selon la forme de réalisation illustrée chacun des stabilisateur **3, 4** est réalisé dans une bandelette de tricot de polyéthylène dont les rangs de mailles sont orientés de manière à être disposés transversalement à l'axe longitudinal de chaque stabilisateur. Chaque bandelette constitutive d'un stabilisateur de suspension est alors rapportée et fixée sur le corps de support au moyen d'une couture **31** sur une patte de fixation **32** offerte par le corps **2**. Pour faciliter la compréhension, la **fig. 2** illustre une vue éclatée de l'implant avant fixation des stabilisateurs de suspension **3, 4** sur le corps **2.** Il est à noter que le corps **2** de l'implant est, selon cet exemple, réalisé dans un tricot à mailles plus lâches et plus extensibles que le tricot constitutif des stabilisateurs de suspension. De plus les stabilisateurs de suspension supérieurs **3** et inférieurs **4** sont selon l'invention indépendants les uns des autres en étant seulement reliés par le corps **2**.

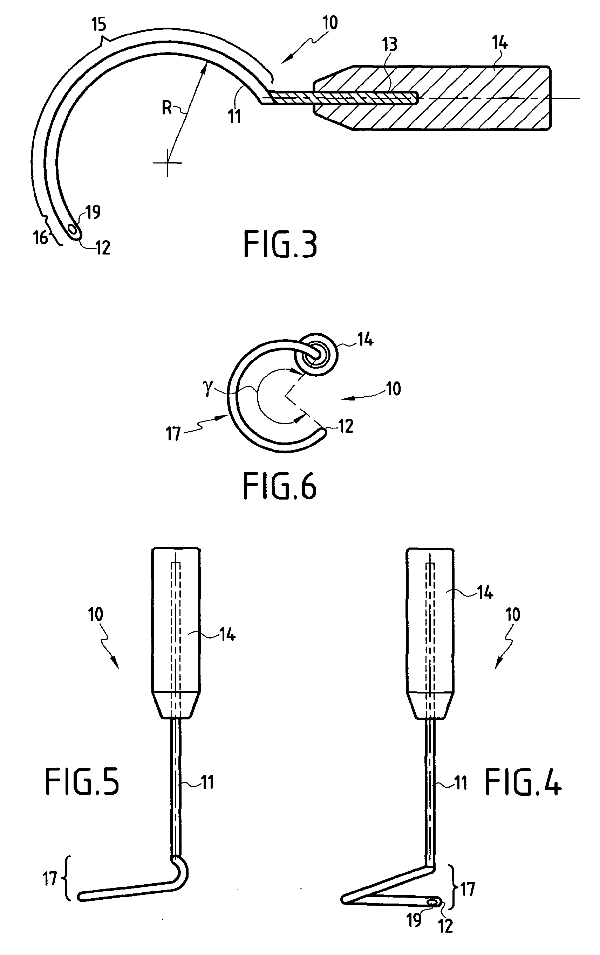

L'implant **1**, tel que décrit précédemment, est destiné à être mis en place au niveau de la cloison recto vaginale d'une patiente. A cet effet, afin de réduire au minimum la dissection de cette région et le traumatisme en découlant, l'invention propose au chirurgien procédant au traitement d'utiliser un ou plusieurs guides perforateurs allongés **10**, tels que ceux plus particulièrement illustrés aux fig. **3** et **4** à **6**.

De manière générale, un tel guide perforateur **10** comprend un corps ou mandrin allongé **11** dont une extrémité **12** est destinée à être introduite dans le corps du sujet à traiter et dont l'autre extrémité **13** est pourvue d'une poignée **14.** Il doit être remarqué que l'extrémité d'introduction **12** est, de préférence, constituée par une pointe mousse, c'est-à-dire une pointe atraumatique qui n'est pas susceptible de blesser ou de couper les tissus dans lesquels elle doit être introduite.

Selon une forme de réalisation illustrée à la **fig. 3,** le guide perforateur **10** présente une forme arquée dans un plan. Cette forme arquée dans un plan est plus particulièrement adaptée pour la mise en place des stabilisateurs de suspension dans les zones transpérinéales et transglutéales. De manière préférée mais non strictement nécessaire la partie arquée du guide perforateur présente alors un rayon de courbure **R** compris entre 30 mm et 60 mm et, de préférence, pour la partie **15** du guide perforateur **10** s'étendant entre la poignée **14** et l'extrémité **12**, compris entre 40 mm et 50 mm, la partie extrême **16** du guide perforateur **10** présentant alors un rayon de courbure variable.

Selon une autre forme de réalisation du guide perforateur **10,** illustrée aux **fig. 4** à **6,** le corps allongé **11** du guide **10** présente une extrémité **17** de forme hélicoïdale, également adaptée pour la mise en place des stabilisateurs de suspension dans les zones supérieure ou inférieure des trous obturés. De manière préférée, l'extrémité distale **17** du guide perforateur présente alors la forme d'une portion de spire hélicoïdale s'étendant sur un angle γ compris entre 180° et 360° et, de préférence, compris entre 255° et 270°. De même, de manière préférée, la spire **17** du guide perforateur présente un rayon de courbure compris entre 20 mm et 40 mm, avec un pas compris entre 15 mm et 25 mm.

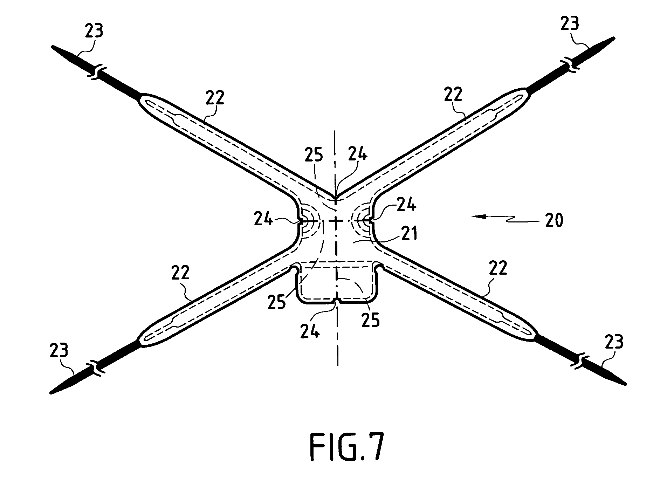

L'implant **1** selon l'invention est, de préférence, disposé de manière à ne présenter aucune tension résiduelle après sa mise en place pour certaines de ses stabilisateurs de suspension au moins. Afin de faciliter ce geste opératoire, l'invention propose dans une variante d'intervention de mettre en oeuvre un introducteur, plus particulièrement illustré à la **fig. 7** et désigné dans son ensemble par la référence **20**.

Cet introducteur présente une structure souple et de forme analogue à celle de l'implant. L'introducteur **20** est, de préférence, réalisé dans un matériau polymère biocompatible, de la famille des matières plastiques à faible coefficient de frottement, tel que, par exemple, le polyéthylène. L'introducteur **20** comprend alors un corps creux **21** définissant une cavité de réception du corps **2** de l'implant **1**. L'introducteur **20** comprend, également, des stabilisateurs tubulaires **22** qui s'étendent à partir du corps creux **21** et qui définissent chacune une cavité de réception d'un stabilisateur de suspension **3, 4** et **5** de l'implant **1**. Chaque bretelle tubulaire **22** présente alors des moyens de traction **23** s'étendant à partir de l'extrémité libre de la bretelle **22** correspondante. Les moyens de traction **23** peuvent être réalisés de toute façon appropriée comme, par exemple, par des systèmes d'accrochage des extrémités des stabilisateurs **22** sur un guide perforateur **10**. Selon l'exemple illustré à la **fig. 7**, les moyens de traction **23** comprennent, pour chaque bretelle **22**, une aiguille souple ou semi-rigide avec une extrémité atraumatique ou mousse. Une telle aiguille peut être réalisée dans le même matériau que le matériau constitutif de l'introducteur **20** ou, plus généralement, dans un matériau choisi dans les polymères synthétiques présentant, de préférence, un faible coefficient de friction.

L'introducteur **20** comprend, enfin, des moyens de découpe **24**, dont la fonction apparaîtra par la suite, du corps creux **21** au moins de l'introducteur **20**. Les moyens de découpe **24** peuvent être alors réalisés de toute façon appropriée et, selon l'exemple illustré, comprennent une série de six ouvertures **24** réalisées à la périphérie du corps creux **21**, entre chacune des stabilisateurs tubulaires **22**, pour permettre le passage d'un outil de coupe pour assurer une découpe du corps creux **21** selon des lignes **25** matérialisées par des traits mixtes à la **fig. 7**.

L'implant **1** se trouve disposé à l'intérieur du corps creux **21** et des stabilisateurs tubulaires **22** en étant, de préférence, libre à l'intérieur de ces derniers, de manière que les efforts exercés sur l'introducteur **20** ne soient pas transmis à l'implant **1** en lui-même.

Le traitement chirurgical de la rectocèle, au moyen d'un implant **1** et de guides perforateurs **10** tels que décrit précédemment, s'effectue de la manière suivante.

La patiente à traiter fait tout d'abord l'objet d'une anesthésie qui peut être générale ou régionale ou, encore, locale, selon les préférences du chirurgien et l'état de santé de la patiente. La position opératoire de la patiente sur la table d'opération sera celle de la chirurgie vaginale habituelle, à savoir fesses de la patiente légèrement en dehors de la table d'opération et cuisses fléchies modérément sur l'abdomen.

Tout d'abord, une mèche rectale est mise en place et il est effectué une injection ischémiante.

Ensuite, intervient une dissection de la zone destinée à recevoir le corps de support **2** de l'implant **1**. Il est exercé une traction sur le col utérin, afin d'exposer le cul de sac vaginal postérieur. Une incision vaginale est pratiquée sur la partie postérieure du col utérin, transversalement sur le versant cervical du cul de sac vaginal bien exposé par la traction. Cette incision peut être qualifiée de rétrocervicale horizontale. La tranche de section vaginale inférieure ainsi réalisée est saisie en totalité au moyen par exemple de trois pinces de Alis qui sont tractées vers le bas en exposant le plan recto-vaginal.

Un décollement recto-vaginal est conduit progressivement en libérant la paroi vaginale postérieure. Ce décollement est arrêté en bas et au milieu au-dessus du cap anal. Le décollement est poursuivi latéralement selon le plan de dissection et au contact du muscle releveur en parcourant, de bas en haut, le faisceau pubo-coccygien, puis le faisceau ilio-coccygien pour arriver au niveau du muscle coccygien et du ligament sacrosciatique. Cette dissection est conduite prudemment en refoulant la graisse péri-rectale et le rectum en dedans. Il est à noter qu'il n'y a alors aucune structure à sectionner et qu'il suffit de refouler en dedans la graisse péri-rectale en restant au contact du plancher musculaire. La méthode la plus pratique, pour effectuer cette dissection, consiste à utiliser un doigt, une compresse et un jeu de valves vaginales étroites et longues (valves de Breisky modifiées), dans la mesure où elles permettent une ouverture progressive sous contrôle de la vue de l'espace sans élargissement excessif permettant l'introduction d'un doigt pour l'exécution de la dissection.

Une fois ce travail de dissection effectué, il est alors possible de procéder à l'introduction de l'implant **1**, soit nu, soit encapsulé dans l'introducteur **20**.

Il est tout d'abord procédé à la mise en place d'un premier stabilisateur de suspension supérieur **3**. A cet effet, un guide perforateur **10** arqué au choix du chirurgien, tel que décrit précédemment en relation avec les **fig. 3** et **4** à **6**, peut être utilisé. Le guide perforateur **10** est alors conduit au travers de la fesse de la patiente par une incision puctiforme située 15 mm environ en arrière du point médian de la ligne s'étendant de l'anus à l'ischion. Un doigt, glissé dans le décollement latéral au contact du ligament sacro-iliaque précédemment exécuté recevra alors la pointe du guide perforateur, de manière à conduire celle-ci dans l'incision vaginale inférieure. Un élément de traction **23** de l'introducteur **20**, en relation avec un stabilisateur de suspension supérieur **3**, est fixé dans un chas **19** offert par l'extrémité du guide perforateur **10** et ce dernier est retiré en sens inverse, de manière à entraîner avec lui la bretelle tubulaire **22** de l'introducteur **20** et le stabilisateur ou bras de suspension supérieure **3** qu'elle contient. La bretelle tractée passe alors au travers du ligament sacrosciatique. Il doit être remarqué qu'en l'absence d'introducteur **20**, l'extrémité distale du stabilisateur de suspension supérieur est directement fixée sur le guide perforateur **10** afin d'être tractée.

Le même geste est effectué pour la mise en place de la seconde bretelle, supérieure **3**. Les stabilisateurs supérieurs **3** ainsi passées pour assurer une suspension transglutéale sont alors mises en attente sur pinces.

Les stabilisateurs de suspension inférieurs **4** sont ensuite passées au travers du muscle puborectal de part est d'autre du canal anal et extériorisées par le même orifice fessier que les stabilisateurs supérieurs **3**.

Une fois les quatre stabilisateurs de suspension **3**, **4** engagées, l'introducteur **20** est découpé de manière à libérer l'implant **1**. Le retrait des différents éléments constitutifs de l'introducteur **20** par des tractions exercées deux à deux sur les stabilisateurs tubulaires **22** opposées permet ainsi de déposer l'implant **1**, sans aucune contrainte, sur ce dernier, de sorte qu'il se trouve dans un état qui pourrait être qualifié de détendu.

Le corps de support **2** de l'implant **1** est alors fixé sur les ligaments utéro-sacrés et le tablier **9** sur la face inférieure du col utérin, par un ou plusieurs et, de préférence, trois points de fil résorbable.

L'incision vaginale postérieure est ensuite suturée par du fil résorbable puis une traction est exercée sur les stabilisateurs de suspension supérieurs **3** traversant la région sacrosciatique afin de remonter en bonne position le dôme vaginal. L'excédent éventuel des stabilisateurs de suspension supérieurs **3** et inférieurs **4** peut alors être sectionné et les orifices fessiers fermés au moyen de points réalisés en fil de suture résorbable.

En fin d'intervention, il est mis en place une mèche vaginale, ainsi qu'une sonde vésicale qui seront retirées quarante-huit heures après l'intervention. Les résidus post-mictionnels étant alors mesurés par sondage, afin de s'assurer que la vidange vésicale est satisfaisante, de manière à autoriser une sortie de la patiente.

L'intervention pour le traitement de la rectocèle aura une durée d'une heure environ et il convient d'adopter une durée d'hospitalisation moyenne de quatre jours. L'activité de la patiente sera limitée pendant un mois et il conviendra d'éviter tout bain pendant cette même période. Enfin il conviendra de prévoir une période d'abstinence sexuelle de six semaines après l'opération.

La technique proposée précédemment permet ainsi de ne traiter que la pathologie, à savoir le déséquilibre de la statique pelvienne et donc de restituer une anatomie la plus normale possible en conservant le schéma corporel de l'individu. Cette technique permet, de manière avantageuse, de conserver les organes sains ou n'influençant pas défavorablement la statique pelvienne. En effet, la pathologie cancéreuse aura été écartée par le bilan pré-opératoire et il sera possible d'assurer après l'intervention chirurgicale une surveillance gynécologique fiable.

Par ailleurs, il existe de très faibles risques de cancer génital pelvien et, de plus, le traitement, proposé par l'invention, ne complique pas un accès ultérieur aux organes génitaux et à la région rectale.

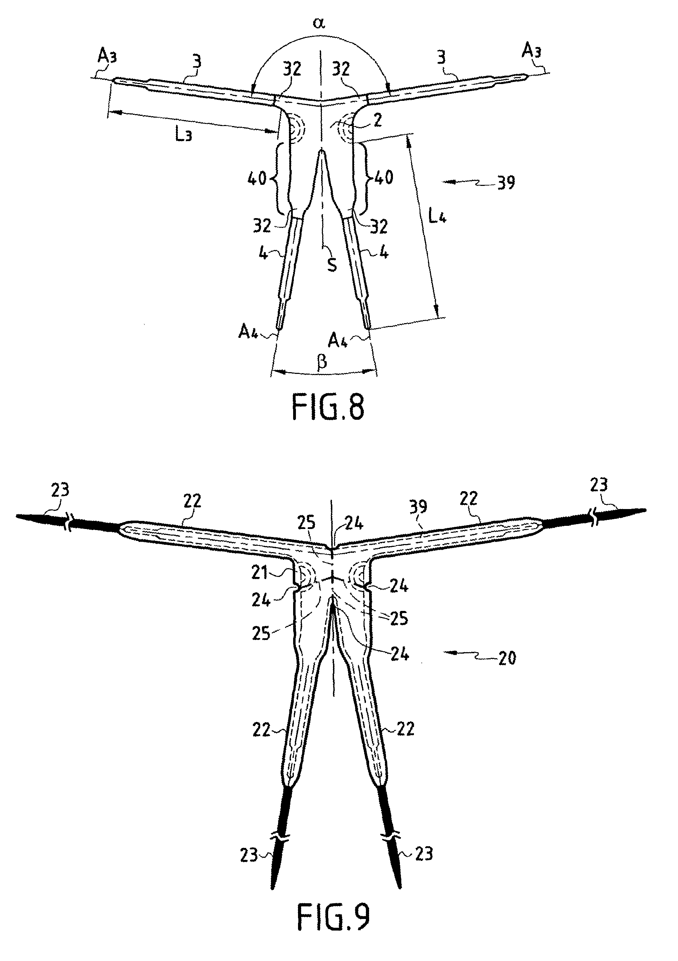

Selon l'exemple de réalisation de l'implant illustré à la **fig. 1,** les stabilisateurs de suspension inférieurs **4** sont fixés sur les pattes **32** qui s'étendent à partir des cotés du corps de support cependant une telle disposition des pattes n'est pas strictement nécessaire à la réalisation d'un implant selon l'invention. Ainsi la **fig. 8** montre un implant **39** conforme à l'invention dont les quatre stabilisateurs de suspension, deux supérieurs **3** et deux inférieurs **4** sont rapportés sur des pattes **32** qui s'étendent à partir des quatre coins du corps de support **2**. Les stabilisateurs de suspension sont ici cousus au niveau de leur bord extrême sur le bord extrême de la patte **32** correspondante.

Selon cette forme de réalisation, les axes **A**_{**3**} des stabilisateurs de suspension supérieure forment un angle α présentant les mêmes caractéristiques que celles de l'implant **1** décrit précédemment, tandis que les axes **A**_{**4**} des stabilisateurs de suspension inférieurs **4** forment un angle β compris de préférence mais non exclusivement entre 10° et 75°.

Par ailleurs, les pattes **32** de fixation des stabilisateurs de suspension inférieurs **4** présentent une forme élargie par rapport au stabilisateur de suspension, de manière à former une zone de suspension pré-rectale **40**.

La **fig. 8** illustre un exemple d'introducteur **20** plus particulièrement adapté à la conformation de l'implant **39**.

L'opération pour le traitement de la rectocèle au moyen de l'implant **39** est conduite comme celle décrite précédemment en ce qui concerne la dissection de la zone recto-vaginale recevant le corps de support **2** et la mise en place les stabilisateurs de suspension supérieurs **3** et inférieurs **4**.

Les parties élargies **40** des stabilisateurs de suspension inférieurs **4** peuvent être fixées par des points de suture résorbables aux muscles releveurs dans la région puborectale.

De même, il peut être envisagé d'assurer le passage des stabilisateurs de suspension inférieurs **4** au travers du périnée. Ce positionnement du hamac pré-rectal en trans-périnéal solidarise, de manière avantageuse, le plan périnéal avec la suspension haute transglutéale, de manière à renforcer les périnées descendants.

Selon les exemples de réalisation des implants décrits précédemment les stabilisateurs de suspension supérieurs **3** et **4** inférieurs s'étendent à partir du corps de l'implant en divergent et confère ainsi à l'implant une forme qui pourrait être qualifié d'étoile. Toutefois cette forme d'étoile n'est strictement nécessaire à la réalisation d'un implant selon l'invention et pour certaine pathologies, il peut apparaître nécessaire de disposer d'un implant dont les stabilisateurs de suspension ne divergent pas mais au contraire dont les extrémités libres des stabilisateurs supérieurs **3** et des stabilisateurs inférieurs **4** sont dirigées vers le bas. Dans un tel cas les axes longitudinaux **A**_{**3**} des stabilisateurs supérieurs forment alors un angle a supérieur à 180° et de préférence supérieur à 200°.

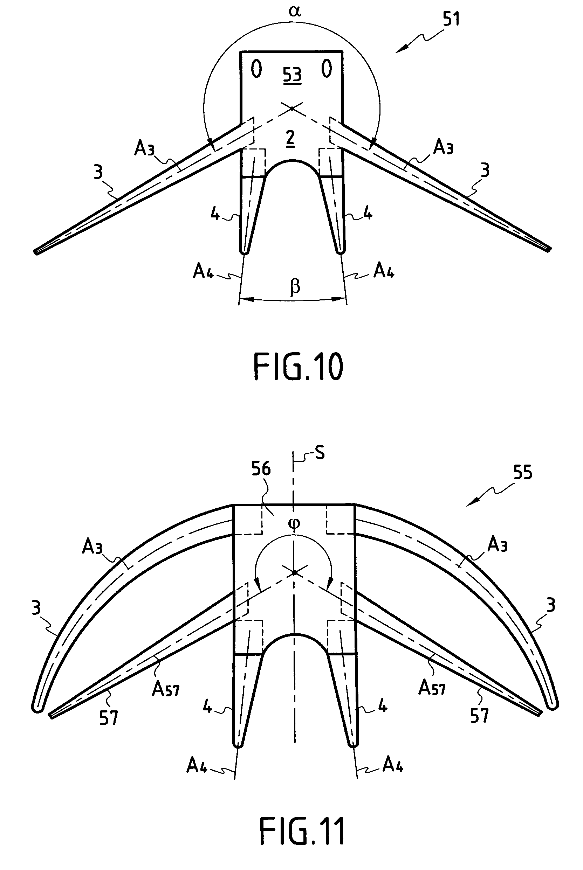

La **fig. 10** montre une telle forme de réalisation d'un implant **51** selon l'invention dont les stabilisateurs de suspension supérieurs **3** s'étendent à partir des cotés du corps **52** de l'implant à distance de son bord supérieur tandis que les deux stabilisateurs inférieurs **4** s'étendent à partir des coins inférieurs du corps de support **52**. Cette configuration particulière définie alors une sorte de tablier supérieur **53** muni de deux orifices **54** pour le passage d'une bandelette rapportée de stabilisation ou pour le passage des stabilisateurs postérieurs d'une prothèse antérieure utilisée en association avec l'implant selon l'invention, une fois que les stabilisateurs de ladite prothèse antérieure ont trans-fixés les ligaments utéro-sacrés.

Par ailleurs, selon la forme de réalisation illustrée à la **fig. 10,** les axes **A**_{**3**} des stabilisateurs de suspension supérieurs **3** forment un angle **α** plus particulièrement compris entre 210° et 260° alors que les axes **A**_{**4**} des stabilisateurs de suspension inférieurs **4** forment un angle **β** inférieur à 45° et de préférence nul.

Il doit être noté que selon cette forme de réalisation le corps **2** deux de l'implant de comprend pas de patte spécifique pour la fixation des stabilisateurs de suspension qui sont ici collés directement sur le corps de support.

La **fig. 11** illustre encore une autre variante de réalisation d'un implant **55** pour le traitement du prolapsus du dôme vaginal et de la rectocèle dont les stabilisateurs de suspension sont tous orientés vers le bas. Selon cette variante, le corps **56** de l'implant présente une forme rectangulaire. Les deux stabilisateurs de suspension supérieurs **3** et les deux stabilisateurs de suspension inférieurs **4** s'étendent alors à partir respectivement des coins supérieurs et inférieurs du corps de support **56**. Selon cet exemple les stabilisateurs de suspensions supérieurs **3** sont arqués et leurs fibres moyennes **A**_{**3**} qui sont l'équivalent des axes **A**_{**3**} dans le cas d'un bras ou d'un stabilisateur rectiligne forment un angle **α** supérieur à 180°. L'angle α est mesuré entre deux tangentes des fibres moyennes des stabilisateurs de suspension supérieurs. Il est à noter que la concavité des stabilisateurs de suspension supérieurs **3** est orientée vers le bas.

Selon la variante de réalisation illustrée **fig. 11**, l'implant **55** comprend en outre des stabilisateurs de suspension moyennes **57** disposées de part et d'autre du plan sagittal **S** et entre les stabilisateurs supérieurs **3** et inférieurs **4.** Les extrémités des stabilisateurs de suspension moyens sont orientées vers le bas et leurs axes **A**_{**57**} forment un angle ϕ de préférence supérieur à 200° et de manière plus particulièrement préférée compris entre 210° et 260°. De plus, les extrémités des stabilisateurs supérieurs **3** et moyens **57** situées d'un même côté du plan sagittal **S** convergent vers un point ou tout au moins sensiblement dans une même direction comme le montre la **fig. 11,** cette caractéristique facilitant la procédure d'implantation comme cela ressort de la suite. Selon cet exemple chacun des bras stabilisateur est soudé directement sur le corps de l'implant.

La mise en place de la prothèse telle qu'illustré à la **fig.11** et décrite précédemment pour le traitement du prolapsus du dôme vaginal et de la rectocèle comprend après une anesthésie de la patiente une phase de dissection. Il est alors utilisé une pince de Muze qui ce une traction sur le col utérin à fin d'exposer le cul-de-sac vaginal postérieur. Une incision vaginale est pratiquée sur la face postérieure du col utérin transversalement sur le versant cervical du cul-de-sac vaginal bien exposé par la traction.

La tranche de section vaginale postérieure est saisie en totalité par 3 pinces d'Alis qui sont tractées vers le bas exposant le plan rectovaginal. Le décollement rectovaginal est conduit progressivement en éversant la paroi vaginale postérieure. Ce décollement est arrêté en bas et au milieu au-dessus du cap anal. Toute dissection du plan recto anal est parfaitement inutile, voir nuisible, car il s'agit d'un plan non anatomique mais créé de toute évidence par la chirurgie. Latéralement le plan de dissection et au contact du muscle releveur parcourant de bas en haut le faisceau pubo-coccygien puis le faisceau ilio-coccygien pour arriver au niveau du muscle coccygien et du ligament sacrosciatique. Cette dissection est conduite prudemment, en refoulant la graisse périrectale et le rectum en dedans. Il n'y a aucune structure à sectionner ; il suffit de refouler en dedans la graisse périrectale en restant au contact du plancher musculaire. Le plus pratique est d'utiliser le doigt, la compresse, et surtout un jeu de deux valves vaginales étroites et longues ; celles-ci ouvrent progressivement sous contrôle de la vue l'espace sans élargissement excessif de l'espace disséqué de quoi glisser un doigt.

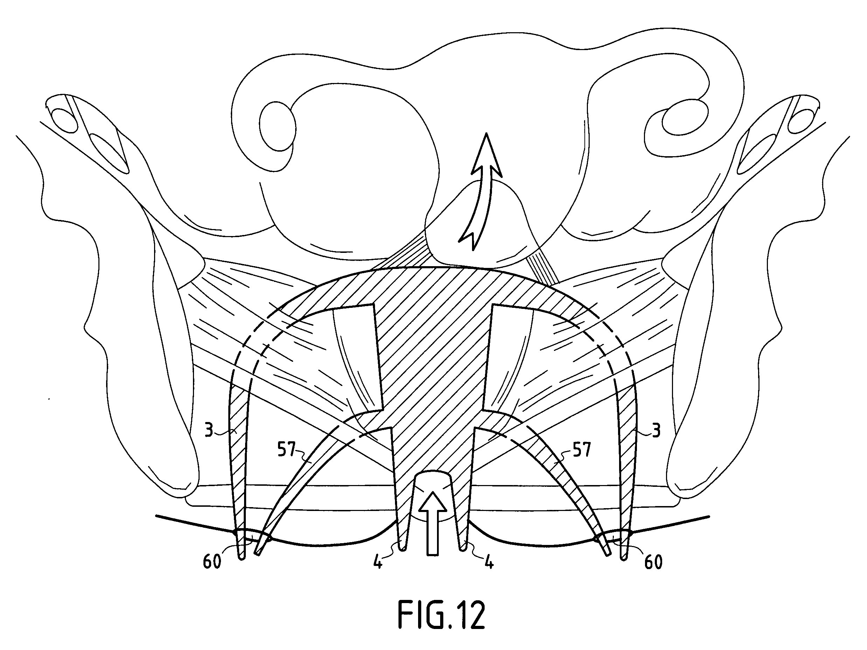

Ensuite un guide perforateur **10** est conduit à travers la fesse par une incision punctiforme **60** située 15 mm en arrière du point médian de la ligne s'étendant de l'anus à l'ischion comme le montre la **fig.12.** Le doigt glissé dans le décollement latéral au contact du ligament sacro-iliaque va recevoir la pointe du guide perforateur **10** et conduire celui-ci dans l'incision vaginale postérieure une extrémité d'un stabilisateur supérieur **3** de l'implant est fixée sur le guide perforateur **10** et tractée à travers le ligament sacrosciatique (suspension sacrosciatique), le même geste est fait de l'autre côté pour l'autre stabilisateur supérieur de l'implant **55**. Le bord supérieur de l'implant est alors suturé par 2 à 4 points à la face postérieure de l'isthme et aux ligaments utérosacrés. Les deux extrémités libres des stabilisateurs supérieurs **3** de l'implant sont ensuite mises en attente sur pince. Les stabilisateurs de suspension moyens **57**, dits de suspension puborectale, sont passés à travers les muscles puborectaux par une voie transpérinéale utilisant le même orifice d'entrée postérieur que pour les stabilisateurs de suspension supérieurs mais avec une orientation en dedans pour émerger au 1/3 moyen de la hauteur de la colpocèle à travers le muscle puborectal. Les stabilisateurs de suspension inférieurs, dits encore périnéaux, sont quand à eux passés de part et d'autre de la fourchette vulvaire grâce à un guide perforateur **10** qu'il est plus prudent d'introduire de dedans en dehors (haut vers le bas) que de bas en haut, car dans ce sens, dans sa course, l'instrument se rapproche du rectum et peut le menacer.

En fin d'intervention, l'incision vaginale postérieure est suturée par un fil résorbable et une traction moyenne est exercée sur les stabilisateurs de suspensions supérieurs et moyens afin de remonter en bonne position le dôme vaginal, et de positionner sans tension excessive le hamac pré rectal.

L'opération s'achève ensuite comme cela à été décrit précédemment.

## Revendications

1. Implant, pour le traitement de la cystocèle, de la rectocèle et/ou du prolapsus du dôme vaginal, présentant une structure mince, souple et comprenant un corps de support (**2**) à partir duquel s'étendent au moins :
■ deux stabilisateurs de suspension supérieurs (**3**),
■ et deux stabilisateurs de suspension inférieurs (**4**), disposés de part et d'autre du plan sagittal (**S**),
**caractérisé en ce que** chaque stabilisateur de suspension (**3, 4, 57**) comprend des moyens aptes à présenter une faible extensibilité parallèlement à son axe longitudinal.

2. Implant selon la revendication 1, **caractérisé en ce que** les stabilisateurs de suspension (**3, 4, 57**) comprennent des moyens aptes à présenter une extensibilité parallèlement à leur axe longitudinal inférieure à l'extensibilité du corps de l'implant selon son axe longitudinal ou son axe transversal.

3. Implant selon la revendication 1 ou 2, **caractérisé en ce que** chaque stabilisateur de suspension (**3, 4, 57**) est rapporté sur le corps de support (**2**).

4. Implant selon la revendication 3, **caractérisé en ce que** le corps de support présente une patte d'attache (**32**) pour chaque stabilisateur de suspension (**3, 4**).

5. Implant selon la revendication 3 ou 4, **caractérisé en ce que** chaque stabilisateur de suspension (**3, 4**) est cousu sur le corps de support.

6. Implant selon la revendication 3 ou 4, **caractérisé en ce que** chaque stabilisateur de suspension est collé sur le corps de support (**2**).

7. Implant selon la revendication 3 ou 4, **caractérisé en ce que** chaque stabilisateur de suspension (**3, 4**) est soudé sur le corps de support (**2**).

8. Implant selon l'une des revendications 1 à 7, **caractérisé en ce que** chaque stabilisateur de suspension (**3, 4, 57**) est réalisé dans un matériau tricoté dont les fils de trame ou les fils de chaîne sont disposés parallèlement à l'axe longitudinal du stabilisateur de suspension.

9. Implant selon l'une des revendications 1 à 8, **caractérisé en ce que** chaque stabilisateur de suspension (**3, 4, 57**) est réalisé dans un tricot peu extensible parallèlement à l'axe longitudinal du stabilisateur de suspension.

10. Implant selon l'une des revendications 1 à 7, **caractérisé en ce que** chaque stabilisateur suspension (**3, 4, 57**) est réalisé dans une nappe de matériau non tissé présentant une résistance à la traction isotrope dans son plan d'extension.

11. Implant selon l'une des revendication 1 à 10, **caractérisé en ce que** chaque stabilisateur de suspension **(3, 4, 57)** est réalisé dans un matériau synthétique biocompatible.

12. Implant selon l'une des revendication 1 à 11, **caractérisé en ce que** chaque stabilisateur de suspension (**3, 4, 57**) est réalisé en matériau biocompatible résorbable ou non.

13. Implant selon l'une des revendications 1 à 12, **caractérisé en ce que** le corps de support (**2**) présente une forme générale sensiblement rectangulaire.

14. Implant selon la revendication 13, **caractérisé en ce que** le corps de support (**2**) présente une longueur (**L**) comprise entre 60 et 90 mm et une largeur comprise entre 40 et 60 mm.
